# EUROPEAN PATENT APPLICATION

(11) **EP 1 992 938 A1**
(43) Date of publication of application: **19.11.2008**
(21) Application number: 07108167.3
(22) Date of filing: 14.05.2007
(51) Int. Cl.: G01N 21/65, G01N 33/543, G01N 33/58

(54) **Improved methods of SE(R)RS detection using multiple labels**

(71) Applicant: Koninklijke Philips Electronics N.V., 5621 BA Eindhoven (NL)
(72) Inventor: The designation of the inventor has not yet been filed
(74) Representative: Van Velzen, Maaike Mathilde

(57) **Abstract**

The present invention relates to methods and devices for sensitive detection of target molecules such as DNA, using surface-enhanced (resonance) Raman spectroscopy (SE(R)RS). The principle of the methods is that the SE(R)RS signal is amplified by using particles with two or more SE(R)RS labels attached thereto in the detection of a target molecule. This increases sensitivity of detection and allows for a more accurate detection. Devices are also provided to practice the methods described herein.

## Description

### FIELD OF THE INVENTION

The present invention relates to methods, kits and tools for the detection and/or quantification of an analyte using surface-enhanced (resonance) Raman scattering (SE(R)RS) with improved accuracy, reproducibility and sensitivity.

### BACKGROUND

The sensitive and accurate detection, either qualitatively or quantitatively, of low concentrations of biomolecules such as proteins, peptides, oligonucleotides, nucleic acids, lipids, polysaccharides, hormones, neurotransmitters, metabolites, etc. has proven to be an elusive goal with widespread potential uses in medical diagnostics, pathology, toxicology, epidemiology, biological warfare, environmental sampling, forensics and numerous other fields such as comparative proteomics and gene expression studies.

A particular example is the detection of DNA e.g. in medical diagnostics (the detection of infectious agents like pathogenic bacteria and viruses, the diagnosis of inherited and acquired genetic diseases, etc.), in forensic tests as part of criminal investigations, in paternity disputes, in whole genome sequencing, etc.

While the identification and/or quantification of a purified sample of a biological analyte can sometimes be performed based on the physicochemical properties of the analyte itself, most detection methods which are capable of identifying and/or quantifying an analyte in a non-purified sample make use of a "probe" which is a known molecule having a strong affinity and preferably also a high degree of specificity for the analyte. Where the analyte is a protein or peptide, these assays are referred to as ligand-binding assays (e.g. immunoassays). Detection of DNA typically makes use of the hybridization of a nucleotide sequence which is specific for the target DNA.

In these probe-based detection assays the analyte-specific probe (or the analyte) is either directly or indirectly labelled with a traceable substance. The detection of the traceable substance (hereafter referred to as "label") bound via the probe to the analyte, is indicative of the presence and/or amount of analyte in the test sample. Detection of the label can be ensured using a variety of different techniques, depending upon the nature of the label employed.

Current detection methods typically involve detection of fluorescently labelled antibodies or oligonucleotide probes that can bind to the analyte or target biomolecule. Cross-reactivity and non-specific binding may complicate fluorescent detection of biomolecules in complex samples. Even where high probe-specificity is obtained, the sensitivity of fluorescent detection is often insufficient to identify low concentrations of biomolecules. Fluorescence spectra contain broad bands typically in the order of multiple nanometers, therefore multiple fluorescent species in solution need multiple excitation wavelengths to discriminate between the broad band spectra.

Surface enhanced (resonance) Raman spectroscopy or SE(R)RS is a technique that is rapidly gaining on fluorescence for detection in view of its impressive sensitivity. Raman spectroscopy utilizes the phenomenon of Raman scattering. When light passes through an optically transparent sample, a fraction of the light is scattered in all directions. Most of the scattered photons are of the same wavelength as the incident light. This is known as Rayleigh scattering. However, a small fraction of the scattered light has a different wavelength and a slight random alteration in phase. The wavelengths of the Stokes (Anti-Stokes) Raman emission spectrum are shifted to longer (or shorter) wavelengths relative to the excitation wavelength. The Raman spectrum is characteristic of the chemical composition and structure of the molecules in a sample with very narrow emission bands, while the intensity of Raman scattering is (often linearly) dependent on the concentration of these molecules. The intrinsically weak Raman scattering can be enhanced by factors of up to 10⁸ or more when a compound is adsorbed on or near structured (roughened) metal surfaces, e.g. solid substrates or nanoparticles of gold, silver, copper and certain other metals, such as noble and alkali metals. The technique associated with this phenomenon is known as surface-enhanced Raman scattering (SERS). The increase in detection sensitivity is more marked the closer the analyte/label sits to the "active" surface. The optimum position is in the first molecular layer around the surface, i.e. within about 30 nm of the surface.

A further 10³-fold increase in sensitivity can be obtained by operating at the resonance frequency of the analyte or, as is more commonly done, making use of a 'SERS-active' substance or dye attached to the analyte (capable of generating a SE(R)RS spectrum when appropriately illuminated), and operating at the resonance frequency of the dye. This is termed "resonance Raman scattering" spectroscopy. The combination of the surface enhancement effect and the resonance effect to give "surface enhanced resonance Raman scattering" or SERRS strongly increases the sensitivity of the method. Compared to fluorescence a SERRS signal can be more easily discriminated from contamination and background due to its sharp vibrational bands instead of broad electronic bands in fluorescence.

Another key advantage of SERRS is the possibility of multiplexing using a single excitation wavelength. Each SERRS-active dye used as a label gives a unique fingerprint which can be recognized in a dye mixture without separation as would be necessary for fluorescence spectroscopy. There are many dyes, either specially designed dyes for SERRS or dyes that were previously used in other detection methods, each with a unique spectrum. SERRS is thus a highly sensitive and specific method for biomolecule detection giving increased sensitivity for the detection of low concentrations of biomolecules. Bioanalytical techniques using SERRS have been demonstrated to allow detection of attomole (10⁻¹⁸ mol) quantities of proteins or DNAs down to femtomole (10⁻¹⁵ mol in 400 µl) concentrations. Single-molecule detection has been reported for rhodamine 6G (R6G), adenine, crystal violet, and other SERRS-active molecules. Raman spectroscopy is applied very broadly, from material analysis in physics to a very wide variety of applications in biology.

Nevertheless, even though very sensitive detection methods such as SERRS are available, reproducible accurate detection of low concentrations of analytes remains a challenge. Especially for biomolecule detection, amplification or purification is often required to obtain detectable concentrations as biomolecules are often present at very low concentrations. Thus, detection of these molecules often requires (extensive) sample preparation time (e.g. DNA amplification by PCR, increasing concentration by purification or dialysis). For these applications, major challenges are to speed up the sample preparation thereby reducing the analysis time, and to make the analysis more sensitive.

Currently, for the identification of a specific target molecule (e.g. DNA sequence) by SE(R)RS, usually a single SE(R)RS probe per target molecule is used to indicate the presence of the target molecule. US2004/0086897 describes particles having bound thereto one or more Raman labels and a specific binding member, and methods of detection using these particles. Briefly, a complex is formed between the particle and the analyte, the complex is bound to a substrate, after which it is stained on the substrate to activate the SERS effect of the Raman label, and the SERS effect is measured. The possibility of using different Raman labels bound to these particles, in combination with particular specific binding members, to allow multiplexing is also described.

### SUMMARY OF THE INVENTION

It is an object of the invention to provide improved SE(R)RS methods, kits and tools. An aspect of the present invention is SE(R)RS methods, kits and tools with increased sensitivity by introducing multiple labels per target molecule. Through the use of multiple optically active labels the signal strength per target molecule can be increased significantly as compared to using a single label per target. This significantly increases the sensitivity of the detection. The multiple-label method is used in combination with Surface-enhanced (resonance) Raman spectroscopy (SE(R)RS), e.g. using SERS active surfaces such as silver or gold nanoparticles or a solid SERS active surface, for analysis of (bio-)molecules. The increase in signal strength results in highly sensitive detection. A higher sensitivity means that lower concentrations of the target molecule can be detected and/or that the analysis time for detecting a specific concentration of analyte can be reduced. For example, fewer purification and/or amplification steps are necessary or amplification steps may even be redundant. Also, increasing the signal-to-noise ratio also makes a higher degree of multiplexing possible.

It is an advantage of the present invention that SE(R)RS-based detection methods can be provided that can be performed quicker and/or in a simpler way, i.e. with fewer steps and/or reagents. More particularly, in methods of the present invention, the readout procedure is quicker. According to one aspect of the invention, methods are provided that take away the need for staining procedures (e.g. silver enhancement procedures using silver ions and a reducing agent like hydroquinone) by providing a SERS active surface for detection.

Particular embodiments of methods of the present invention also allow a faster and more homogenous diffusion of reagents prior to detection, which furthers the reproducibility of the assay. According to one particular aspect of the invention methods are provided that can be performed completely in solution, in the absence of a solid surface.

According to an aspect of the present invention, methods and tools are provided for sensitive detection of a target molecule using surface-enhanced (resonance) Raman spectroscopy (SE(R)RS), whereby the target is captured by a complex comprising two or more labels, thereby amplifying the signal of the target.

According to another aspect of the invention, methods are provided wherein the target is sandwiched between a particle comprising multiple labels and a manipulatable particle. More particularly, according to this aspect, methods are provided for sensitive detection of a target molecule using surface-enhanced (resonance) Raman spectroscopy (SE(R)RS), having the steps of:
(a) providing labelled particles carrying one or more first target-specific probes capable of binding specifically to part of the target molecule, wherein the labelled particles comprise two or more SE(R)RS labels,
(b) providing manipulatable particles carrying one or more second target-specific probes capable of binding specifically to another part of the target molecule,
(c) allowing the target molecule to be bound, in random order, by both the one or more first target-specific probes of the labelled particles and the one or more second target-specific probes of the manipulatable particles, thereby obtaining a "sandwiched" target molecule,
(d) separating the "sandwiched" target molecule from the unbound labelled particles;
(e) optionally, releasing the SE(R)RS labels from the manipulatable particles;
(f) providing a SERS active surface to which the SE(R)RS labels can adsorb;
(g) allowing the two or more SE(R)RS labels to adsorb to the SERS active surface; and
(h) measuring the SE(R)RS spectrum of the two or more SE(R)RS labels adsorbed to the SERS active surface.

According to particular embodiments of methods of the present invention, the particles comprising the two or more SE(R)RS labels are SE(R)RS labelled nanoparticles carrying the SE(R)RS labels on their surface. More particularly the SE(R)RS labelled nanoparticles have a noble metal surface such as a gold surface. According to a further particular embodiment, these nanoparticles are gold nanoparticles. According to further particular embodiments, the SE(R)RS labelled nanoparticles have a silver surface. In a further embodiment, the nanoparticles are silver nanoparticles.

Methods of the present invention are based on detection of SE(R)RS labels. SE(R)RS labels on the particles may be attached to the probes, or attached directly or indirectly to the particle surface, or a combination thereof. According to a particular embodiment, SE(R)RS labels are attached to the first target-specific probes. According to a further embodiment, SE(R)RS-labelled probes are bound covalently on the particle surface. In a further specific embodiment, covalent binding between the probes and the particle surface is via a thiol group on the probe.

In particular embodiments, labelled particles used in the methods of the invention carry one or more SE(R)RS labelled probes. According to a further particular embodiment, each probe carries more than one SE(R)RS label.

According to another embodiment, methods are provided wherein SE(R)RS labels are attached directly to the particle surface. Typically the particle is a nanoparticle and SE(R)RS labels are attached to the nanoparticle surface via covalent binding. More in particular, this covalent binding may be via a thiol group. According to another specific embodiment, a SE(R)RS label has a benzotriazole moiety for covalent attachment, and attachment of the SE(R)RS label to the nanoparticle surface is via the benzotriazole moiety.

Methods according to the above aspect of the invention involve the use of manipulatable particles, which can be used to ensure the separation of a SE(R)RS label bound to the target from an unbound SE(R)RS label. Different embodiments of manipulatable particles are envisaged in the context of the present invention. According to a particular embodiment, manipulatable particles are magnetic or magnetizable particles. Alternatively, manipulatable particles are dielectric particles, which can be manipulated in an electric field. According to yet another particular embodiment, the manipulatable particles are manipulatable by optical means (such as optical tweezers). In yet another specific embodiment, particles are manipulatable in that they can be manipulated based on their physical properties such as by using a mechanical filter (separation by size).

According to particular embodiments of the invention, detection methods comprise the step of releasing the labels from the manipulatable particles. In particular embodiments the step of releasing the SE(R)RS labels from the manipulatable particles is done by releasing the SE(R)RS labels from the (nano)particles. SE(R)RS labels may be released as such, or still be attached to a target-specific probe or a linker molecule. According to a more particular embodiment, the release of the SE(R)RS labels from the nanoparticles is performed by using dithiothreitol (DTT). According to an alternative particular embodiment, release of the SE(R)RS labels is performed with an enzyme. According to other specific embodiments release of the SE(R)RS labels is done by changing the pH. Particular embodiments of methods of the invention encompass a step of releasing labels from the manipulatable particles comprising releasing the labelled particles from the target molecule, and/or by releasing the target molecule and labelled particle from the manipulatable particle.

According to one embodiment of methods of the present invention, the SE(R)RS active surface provided for detection is a metal surface, more in particular a noble metal surface. According to a further specific embodiment, the SE(R)RS active surface is a gold or silver surface.

The SERS active surface used in particular embodiments of the methods of the present invention may be a solid substrate, or provided in particulate form (specifically in solution, e.g. a colloidal suspension). According to a specific embodiment, a SERS active surface is a solid metal substrate.

According to another embodiment, a SERS active surface is provided in the form of nanoparticles that are aggregated before the measuring step. According to a particular embodiment, these nanoparticles are metal particles, more in particular particles of a noble metal. According to a further particular embodiment, the nanoparticles with a SE(R)RS active surface are silver particles. According to an alternative particular embodiment, the nanoparticles with a SERS active surface are gold particles.

According to some specific embodiments of the methods of the invention, the SE(R)RS labelled particles provided in step (a) are nanoparticles comprising a SERS active surface and these labelled particles provide at least part of the SERS active surface of step (f).

According to a further embodiment of methods of the invention, the SE(R)RS labelled particles provided in step (a) are nanoparticles comprising a SERS active surface and the methods comprise the step of releasing the SE(R)RS labels from the target molecule by releasing the SE(R)RS labelled nanoparticles from the target molecule and/or from the manipulatable particles.

In those embodiments of the methods of the invention where the SE(R)RS labelled nanoparticles are released from the target molecule for detection, release of the nanoparticles, e.g. from the target molecule may be ensured through heating. In alternative embodiments, release of the nanoparticles from the target molecule or from the manipulatable particles is ensured by an enzyme. Another alternative embodiment provides release of the nanoparticles from the target molecule by changing the salt concentration or the pH of the environment.

In particular embodiments of methods of the invention where the SE(R)RS labelled nanoparticles are released from the target molecule and/or from the manipulatable particles before detection, the methods comprise a further step of adding unlabelled nanoparticles with a SERS active surface to the reaction mixture. These unlabelled nanoparticles may be of the same material as the SE(R)RS labelled nanoparticles. In these embodiments of the invention, typically an aggregating agent will be provided to ensure aggregation of the nanoparticles comprising a SERS active surface. In a more particular embodiment, the aggregating agent is a polyamine. According to a further specific embodiment, the polyamine is spermine. According to an alternative embodiment, the aggregating agent is a salt.

According to particular embodiments, methods of the invention comprise an additional step, wherein the SE(R)RS spectrum or the intensity of the SE(R)RS spectrum is correlated to the presence of the target molecule. Where methods of the invention are used in multiplex assays (i.e. to detect more than one target molecule simultaneously), a different SE(R)RS label is used for each target and appropriate target-specific probes are used. The intensity of each of the SE(R)RS spectra is then correlated with the presence of the corresponding target molecule in the sample. Typically, this is done by using distinct SE(R)RS labels for each target molecule.

According to one aspect of the invention, methods are provided for detection of a particular target with increased sensitivity. In this aspect, methods of the invention make use of labelled particles comprising two or more SE(R)RS labels having identical SE(R)RS spectra.

According to another aspect of the invention, methods are provided for detection of a particular target with increased accuracy by measurement of multiple features in the spectrum, i.e. distinguishing the presence of different spectra. In this aspect, methods of the invention make use of labelled particles comprising two or more SE(R)RS labels with different SE(R)RS spectra. The detection step in the methods according to this aspect involve detecting the SE(R)RS spectrum of each of the different SE(R)RS labels used in the methods.

Methods of the present invention encompass the generation of a complex, whereby the target is sandwiched between a first and a second target-specific probe. According to specific embodiments of the invention, the first and second target-specific probes are independently selected from the group consisting of DNA, RNA, PNA, polypeptides, antibodies, antigens, carbohydrates, proteins, peptides, drugs, viruses, polysaccharides, lipids, lipopolysaccharides, glycoproteins, lipoproteins, nucleoproteins, nucleic acids, oligonucleotides, immunoglobulins, albumins, hemoglobins, cell-surface molecules, microorganisms, fragments, portions, components or products of microorganisms, and small molecules, i.e. molecules smaller than 20 kDa.

Methods of the present invention are envisioned to be suitable for the detection of different types of targets, whereby the nature of the target is not critical. In particular embodiments of the methods of the invention, the target molecule to be detected is a nucleic acid, and both the first and second target-specific probes are oligonucleotides. Specific binding of the probes to the target molecule is typically through hybridisation.

In further aspects of the invention, devices and systems are provided suitable for carrying out the methods of the invention. According to one embodiment, such a device is a disposable cartridge for use in a system for detecting the presence and/or quantity of a target molecule in a sample, which comprises:
(a) a contacting container which can be subjected to a separation force such that manipulatable particles can be moved from a first zone to a second zone within the contacting container,
(b) a detection window placed such that detection occurs in one of both zones of the contacting container,
(c) means for ensuring the provision of fluids from sources to the contacting container; and
(d) optionally, a set of sources comprising:
   - at least one source of labelled particles carrying one or more first target-specific probes capable of binding specifically to part of the target molecule, wherein the labelled particles comprise two or more SE(R)RS labels
   - at least one source of manipulatable particles carrying one or more second target-specific probes capable of binding specifically to another part of the target molecule, and
(e) at least one source of additives to be used in the detection.

According to particular embodiments, the cartridges contain one or more additional sources, such as a source of sample containing the target molecule or suspected of containing the target molecule and/or at least one source of internal standard reagents.

According to particular embodiments of the cartridges provided herein, the at least one source of additives of a cartridge comprises one or more sources selected from the following: nanoparticles with a SERS active surface, aggregating agents, enzymes, buffers, and dithiothreitol (DTT).

According to a particular embodiment, the separation force to which the cartridge can be subjected is a magnetic field. According to another particular embodiment, the separation force is the application of an electric field. According to another specific embodiment, the separation force is an optical separation force, such as provided by optical tweezers. According to yet another alternative embodiment, the separation force is a mechanical separation force, e.g. based on size, e.g. by application gravity, i.e. gravimetric separation.

The detection window in the devices and cartridges according to the invention can also be a detection region or detection zone, like e.g. in lateral flow devices.

Optionally, the cartridges of the present invention comprise a contacting container comprising a solid SERS active surface placed such that the detection window allows detection of SER(R)S labels bound to the SERS active surface. Optionally the cartridges comprise a separate detection container comprising the detection window and the SERS active surface.

The cartridges provided in the present invention may be used in a system for detection of a target molecule. Accordingly a further aspect of the invention, provides systems for detecting the presence or amount of a target molecule, comprising:
(a) means for contacting a sample containing the target molecule or suspected of containing the target molecule with:
   - at least one source of labelled particles carrying one or more first target-specific probes capable of binding specifically to part of the target molecule, wherein the labelled particles comprise two or more SE(R)RS labels;
   - at least one source of manipulatable particles carrying one or more second target-specific probes capable of binding specifically to another part of the target molecule;
(b) means for applying a magnetic field;
(c) a detection container, optionally integrated in the contacting means (a); and
(d) means for detecting the signal generated by the two or more SE(R)RS labels.

According to a particular embodiment, the means for detecting (d) has at least the following components: a light source, a filter and a detection means.

According to another embodiment, the system may further have means for processing the signal detected with the means for detecting.

According to another aspect of the invention, methods and devices are provided that use a washing step rather than a magnetic separation step for the separation of bound and unbound labelled particles. The principle of amplifying the SE(R)RS signal with multiple labels, however, remains the same.

Thus, according to this aspect, methods of sensitive detection of a target molecule are provided, using surface-enhanced (resonance) Raman spectroscopy (SE(R)RS), comprising the steps of:
(a) providing labelled particles carrying one or more first target-specific probes capable of binding specifically to part of the target molecule, wherein the labelled particles comprise two or more SE(R)RS labels,
(b) providing a SERS active solid surface carrying one or more second target-specific probes capable of binding specifically to another part of the target molecule,
(c) allowing the target molecule to be bound, in random order, by both the first target-specific probes of the particles and the second target-specific probes of the solid surface, thereby obtaining a "sandwiched" target molecule,
(d) separating the "sandwiched" target molecule from the unbound labelled particles by washing away the unbound labelled particles, and
(e) measuring the SE(R)RS spectrum of the two or more SE(R)RS labels bound to the solid surface.

According to a particular embodiment of methods according to this aspect of the invention, the SERS active solid surface is a solid metal substrate, more in particular a substrate of a noble metal, even more in particular a (roughened) silver or gold substrate or surface. In a further specific embodiment, SERS active nanoparticles can also be added to enhance the signal, more in particular SERS active nanoparticles of the same material as the SERS active solid surface.

In addition; a disposable cartridge is provided for use in a system for detecting the presence and/or quantity of a target molecule, comprising:
(a) a set of sources comprising:
   - at least one source of (nano)particles carrying one or more first target-specific probes capable of binding specifically to part of the target molecule, wherein the (nano)particles comprise two or more SE(R)RS labels;
   - at least one source of buffer;
(b) a detection container comprising a SERS active solid surface carrying one or more second target-specific probes capable of binding specifically to another part of the target molecule; and
(c) means for ensuring the provision of the fluids from the sources to the detection container.

According to further embodiments, a cartridge may contain one or more additional sources. According to one such embodiment, a cartridge further contains a source of sample containing the target molecule or suspected of containing the target molecule.

In accordance with the embodiments of the methods of the invention described above, systems are provided for detecting the presence or amount of a target molecule, optionally in a removable or disposable cartridge, which make use of a solid SERS surface. Such a system according to the invention typically comprises the following components:
(a) means for contacting, optionally within a removable cartridge, a sample containing the target molecule or suspected of containing the target molecule with
   - at least one source of particles carrying one or more first target-specific probes capable of binding specifically to part of the target molecule, wherein the particles comprise two or more SE(R)RS labels;
   - a SERS active solid surface carrying one or more second target-specific probes capable of binding specifically to another part of the target molecule;
(b) means to ensure washing of SERS active solid surface; and
(c) means for detecting the signal generated by the two or more SE(R)RS labels on said SERS active solid surface.

In addition, the present invention provides disposable cartridges for use in the systems of the invention described above. Accordingly, the invention provides disposable cartridges for use in a system for detecting the presence and/or quantity of a target molecule, comprising:
(a) a detection container comprising a SERS active solid surface optionally carrying one or more second target-specific probes; and
(b) means for ensuring the movement of the fluids from different sources to the detection container, and optionally,
(c) a set of sources comprising:
   - at least one source of labelled particles carrying one or more first target-specific probes capable of binding specifically to part of the target molecule, wherein the labelled particles comprise two or more SE(R)RS labels;
   - at least one source of buffer.

Optionally, cartridges of the present invention further comprise a source of sample containing the target molecule or suspected of containing the target molecule.

### DETAILED DESCRIPTION

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### BRIEF DESCRIPTION OF THE FIGURES

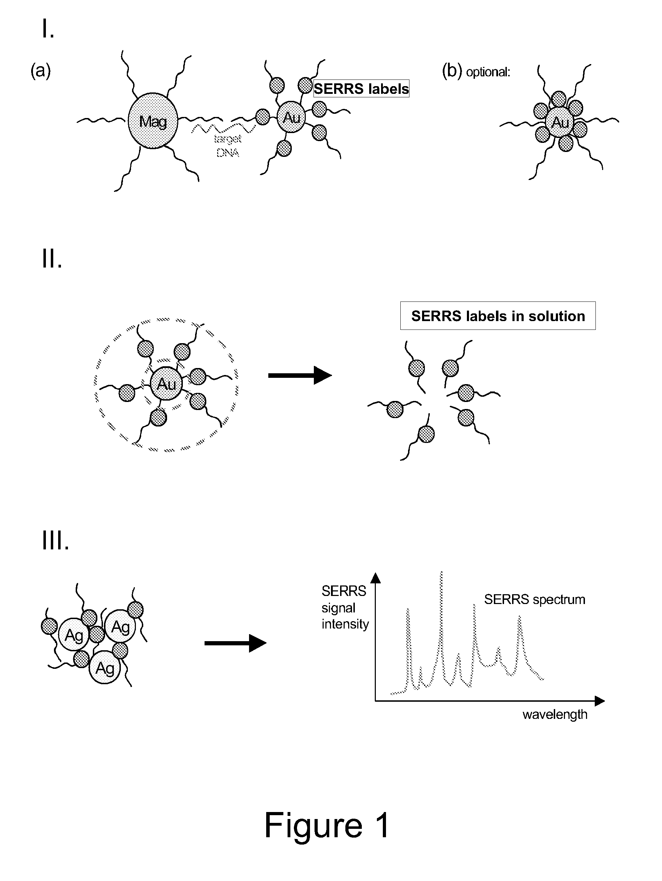

Figure 1 is an illustration of one embodiment of the invention, using gold particles as SE(R)RS labelled nanoparticles with one or more first target-specific probes and silver nanoparticles (or a solid surface) for detection. **I (a):** 'sandwiched' target molecule: a target molecule (here DNA) is hybridized to a gold nanoparticle (Au) carrying a number of first target-specific probes that are labelled with a SE(R)RS label, and a magnetic particle (Mag) with a multiplicity of second target-specific probes. **(b):** an alternative version of the gold nanoparticle (Au), carrying a number of non-labelled first target-specific probes, and SE(R)RS labels on the nanoparticle surface. **II:** illustration of the release from the labels from the nanoparticle by releasing the probes from the particle (after a magnetic separation step). Releasing typically occurs via DTT or an enzyme. **III**: Detection of the SE(R)RS labels (still attached to the probes) by measuring their SE(R)RS spectrum. Silver nanoparticles (Ag) are used as SERS active surface (in combination with an aggregating agent).

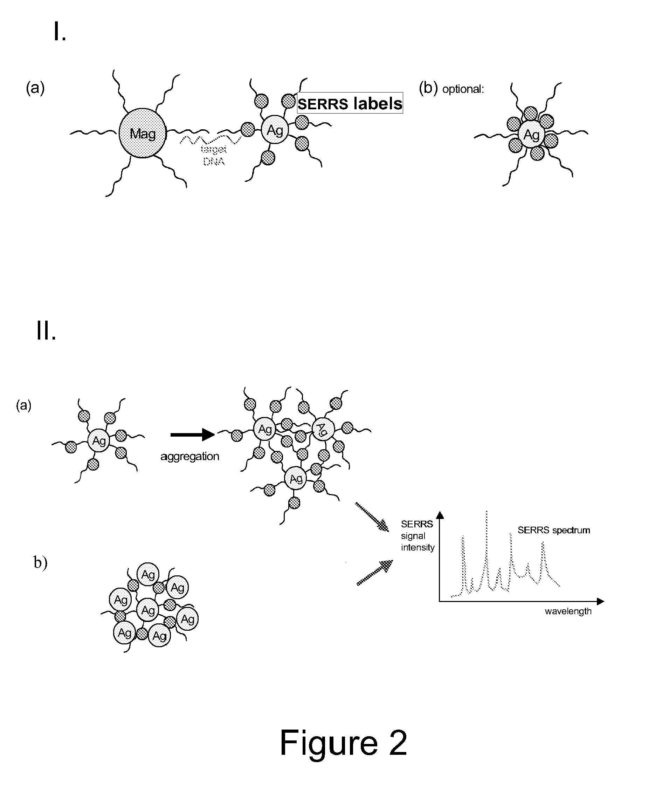

Figure 2 is an illustration of one embodiment of the invention, using silver particles as SE(R)RS labelled nanoparticles and simultaneously as SERS active surface. **I (a):** 'sandwiched' target molecule: a target molecule (here DNA) is hybridized to a silver nanoparticle (Ag) carrying a number of first target-specific probes that are labelled with a SE(R)RS label, and a magnetic particle (Mag) with a multiplicity of second target-specific probes. **(b):** an alternative version of the silver nanoparticle (Ag), carrying a number of non-labelled first target-specific probes, and SE(R)RS labels on the nanoparticle surface. **II:** The SE(R)RS labelled nanoparticles are released from the sandwiched target molecule (e.g. via heat or salt denaturation), after which the SE(R)RS labels can be detected. **(a):** Detection of the SE(R)RS labels (still attached to the nanoparticles) by measuring their SE(R)RS spectrum. The SE(R)RS labelled silver nanoparticles are allowed to aggregate with each other (using an aggregating agent), the particles serve as SERS active surface. **(b):** Detection of the SE(R)RS labels (still attached to the nanoparticles) by measuring their SE(R)RS spectrum. Unlabelled silver nanoparticles (colloids) are added together with an aggregating agent. Both SE(R)RS labelled and unlabelled silver nanoparticles are allowed to aggregate with each other, the particles serve as SERS active surface.

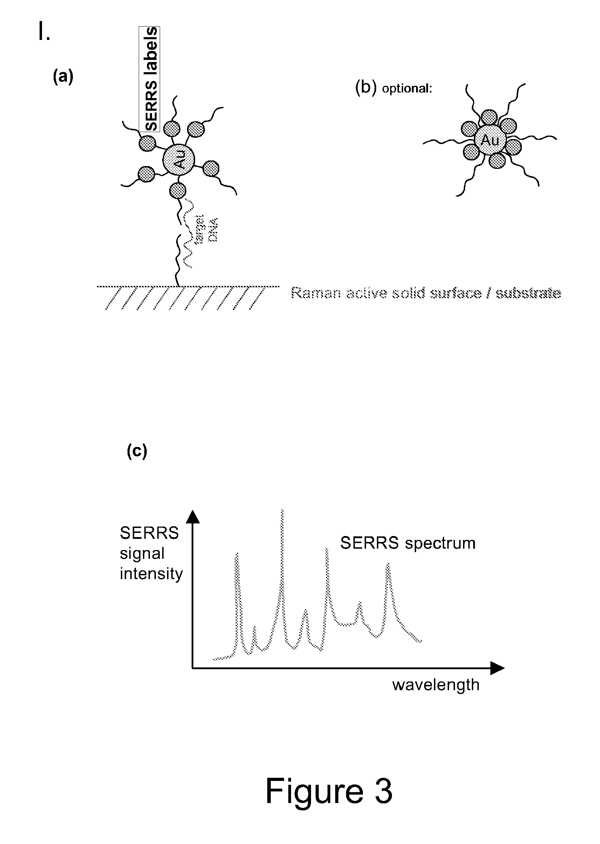

Figure 3 is an illustration of one embodiment of the invention, using a solid SERS active surface instead of manipulatable particles for separation. **I (a):** 'sandwiched' target molecule: a target molecule (here DNA) is hybridized to a gold nanoparticle (Au) carrying a number of first target-specific probes that are labelled with a SE(R)RS label, and a solid SERS active surface carrying one or more second target-specific probes. **(b):** an alternative version of the gold nanoparticle (Au), carrying a number of non-labelled first target-specific probes, and SE(R)RS labels on the nanoparticle surface. **(c):** After washing away the unbound components, the SE(R)RS spectrum of the labels can be measured using the solid surface as SERS active surface.

In the different Figures, the same reference signs refer to the same or analogous elements.

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. These definitions should not be construed to have a scope less than understood by a person of ordinary skill in the art.

### DEFINITIONS

The term 'target molecule', as used herein, refers to the substance to be detected and/or quantified in the methods of the present invention. A non-limiting list of examples of target molecules envisaged by the present application is provided in the description.

The term 'SE(R)RS' as used in the application refers to surface-enhanced (resonance) Raman spectroscopy. A 'SE(R)RS label' as used throughout the application includes any label, dye or other SERS-active substance capable of generating a SE(R)RS signal when appropriately illuminated. A SE(R)RS label thus may e.g. be a Raman label. A SE(R)RS label includes both fluorescent and non-fluorescent labels or dyes. A non-limiting list of examples of labels envisaged for use in the methods of the present invention is provided in the description. The label can be attached to a probe, attached to a nanoparticle, or in specific embodiments, it can be attached to a target molecule.

The term 'SE(R)RS spectrum' refers to the SE(R)RS signal obtained when a SERS-active substance (such as a SE(R)RS label) is appropriately illuminated or excited. This spectrum can be measured at a specific wavelength or frequency (e.g. a resonance frequency of a SE(R)RS label) or at several or multiple wavelengths (e.g. resonance wavelengths of a dye or several dyes) or across a range of wavelengths, and is specific for the SE(R)RS label. Typically, the incident light is of only one wavelength, while the outgoing (scattered) light is measured across a whole range of wavelengths.

The term 'SERS active surface', as used herein refers to a surface capable of enhancing Raman scattering. Such a surface may be used both for SERS or SERRS detection. Typically, a SERS active surface is made of metals, more particularly noble metals such as silver or gold although other materials (copper, platinum, nickel, aluminium, certain alkali metals such as lithium) may be employed as well. The SERS active surface can e.g. be provided as a solid substrate, or in (colloidal) solution in the form of nanoparticles. The solid substrate can take different forms such as a flat sheet, a globular particle, the surface of a container, a tip, or any other shape. The SERS active surface can be the top/outer layer of a structure whereby the nature of the SERS active surface is identical or different from the underlying material (such as e.g. a silver film on a silicone base).

The term 'target-specific probes' as used in the application refers to molecules capable of specifically binding to (part of) a target molecule. Their nature thus may depend on the nature of the target molecule to be detected (e.g. a nucleic acid probe to detect DNA or RNA, an antibody to detect protein or DNA). Typically, a target-specific probe is one member of a specific binding pair and (part of) the target molecule is the other member of that binding pair. A non-limiting list of examples of target-specific probes is included in the specification.

The term "manipulatable particle" in the context of the present invention relates to a particle that can be manipulated by application of an external force or field (e.g. electric or magnetic force, electromagnetic forces such as provided by a light beam or optical tweezers, mechanical forces such as by mechanical filtering, ...).

An 'aggregating agent' is a term used in the application to refer to compounds that are capable of promoting the aggregation of nanoparticles. Typical examples of aggregating agents are polyamines, in particular spermine, but other aggregating agents may be used as well (e.g. salts). A non-limiting list of suitable examples of aggregating agents is included in the specification.

The present invention relates to methods of sensitive detection of a target molecule using surface-enhanced (resonance) Raman spectroscopy (SE(R)RS). More particularly, methods are provided whereby the target is detected making use of particles which comprise multiple SE(R)RS labels, thereby amplifying the signal of the target. More particularly, methods are provided which are based on forming a "sandwiched" target molecule, i.e. a target molecule which is bound by two different target-specific probes simultaneously, one of which ensures the binding of the target to the particle comprising multiple labels.

According to one aspect of the invention, methods are provided wherein the target molecule is bound to both a particle comprising multiple labels and a manipulatable particle. More particularly, these methods comprise the steps of:
(a) providing labelled particles carrying one or more first target-specific probes capable of binding specifically to part of the target molecule, wherein the labelled particles comprise two or more SE(R)RS labels,
(b) providing manipulatable particles carrying one or more second target-specific probes capable of binding specifically to another part of the target molecule;
(c) allowing the target molecule to be bound, in random order, by both the one or more first target-specific probes of the labelled particles and the one or more second target-specific probes of the manipulatable particles, thereby obtaining a "sandwiched" target molecule, and
(d) separating the "sandwiched" target molecule from the unbound labelled particles;
   According to this aspect of the invention, manipulatable particles (e.g. magnetizable, magnetic or (di)electric particles) are used to ensure separation of the labelled particles bound to the target and the labelled particles which have not bound to the target. In the following steps, detection of the labels is ensured. These steps comprise
(e) optionally, releasing the SE(R)RS labels from the target molecule;
(f) providing a SERS active surface to which the SE(R)RS labels can adsorb;
(g) allowing the SE(R)RS labels to adsorb to the SERS active surface; and
(h) measuring the SE(R)RS spectrum of the SE(R)RS labels adsorbed to the SERS active surface.

Accordingly, the SE(R)RS labels are allowed (either actively or passively) to adsorb to a SERS active surface for detection of their SE(R)RS spectrum.

Not all steps need necessarily to take place in the order that they are listed above: steps (a) to (c) may be moved around, but take place before step (d). Step (e), (g) and (h) are typically performed in that order, after step (d). Step (f), however, can be moved forward, e.g. between step (d) and (e), or even before step (d). In one particular embodiment, step (f) may, in whole or in part, be incorporated in step (a). In this particular case, the particles are nanoparticles having a SERS active surface and at least part of these particles form the SERS active surface to which the SE(R)RS labels can adsorb (described in more detail below).

Thus, in practice, the step of providing the particles (a) and providing the manipulatable particles (b) may take place in the order provided above, the reverse order, or may be simultaneous. Accordingly, in step (c) the target molecule can be bound first by the probe on the labelled particle, first by the probe on the manipulatable particle or simultaneously by both probes. The target molecule may even be bound by one of the probes (i.e. part of step (c)) prior to providing the particles with the other probe.

Similarly, the SERS active surface may be provided after the SE(R)RS labels have been released from the target molecule, prior to the release of the SE(R)RS labels or simultaneously with the release of the SE(R)RS labels from the target molecule. According to a particular embodiment, the SERS active surface is already present prior to or during the separation step in (d) (e.g. by moving the "sandwiched" target molecule to a container with a SERS active surface, or by incorporating step (f) at least partly in step (a), as described above).

Methods of the present invention are typically performed for detecting the presence and optionally the quantity of the target molecule in a sample. Accordingly, the methods of the invention are typically carried out on a sample containing the target molecule, or suspected of containing the target molecule. In particular embodiments the step of allowing the target molecule to be bound by the one or more first target-specific probes of the labelled particles and the one or more second target-specific probes of the manipulatable particles is ensured by contacting the sample with the first and second target-specific probes. Alternatively, prior to detection, the target can be enriched or purified from the sample. According to the latter embodiments, the methods of the invention encompass a step of 'providing the target molecule' (i.e. by amplification, separation, purification etc from the sample). This step of providing the target molecule may be prior to steps (a) and (b), simultaneous with (a) and/or (b), after steps (a) and (b), after (a) but before (b), or after (b) but before (a). Again, the order of these steps is not critical, and part of step (c) (the binding of the first or second target-specific probe) thus may take place before step (b) or (a), respectively.

Target molecules envisaged for detection in any of the methods of the present invention in principle include any substance, and in particular substances for which two target-specific probes binding to different parts of the target molecule are available or can be prepared. Target molecules for which such an independent couple of target-specific probes can not be prepared can however also be detected using the methods of the invention. For instance, such molecules can be conjugated with or linked to or attached to a tag (or two tags, depending on the target molecule) for which a target-specific probe exists or can be prepared. More particularly, where the target is a DNA molecule, it can be envisaged that one or more tag DNA sequences are attached to the target by PCR.

Typical examples of types of target molecules envisaged to be detected in the context of the present invention include, but are not limited to, an antibody, an antigen, a hapten, a receptor, a ligand, a protein, a peptide, a polypeptide, a nucleic acid, a membrane or membrane fraction, a lipid, a membrane-protein complex, a carbohydrate, a virus, a bacterium, a cell or macromolecule or molecular complex, a small molecule (i.e. a molecule smaller than 20 kDa), a hormone, a steroid, a vitamin, a drug, a cytokine, a cell-surface molecule, disease-associated epitopes within a protein or peptide (e.g. an epitope found only on a tumor-specific protein), and so on. It is possible that the target molecule is part of a larger molecule or complex, e.g. a protein conjugated with a hapten or a polypeptide fusioned to a nucleic acid. Thus, molecules or complexes comprising a target molecule are also envisaged for detection using the methods of the invention.

According to a particular embodiment of the tools and methods of the present invention, the target molecule to be detected is a nucleic acid. Examples of nucleic acids include genes, viral RNA, DNA or fragments thereof, bacterial DNA, RNA or fragments thereof, fungal DNA or RNA or fragments thereof, mammalian DNA, cDNA, mRNA, RNA or fragments thereof, peptide nucleic acid (PNA), oligonucleotides, synthetic oligonucleotides, modified oligonucleotides, single-stranded and double-stranded nucleic acids, natural and synthetic nucleic acids, or molecules containing such nucleic acids.

According to another particular embodiment, the target molecule is a protein or peptide.

Methods of the invention allow detection with improved sensitivity such that pre-treatment of the sample may not be necessary. Advantages of performing the methods without amplification of the target include that the target molecule can be more easily quantified, as the intensity of the resulting SE(R)RS spectrum is directly correlated to the amount of target molecule present. Another advantage of omitting amplification or other types of processing is that the methods can be performed faster and simpler. Indeed, in many detection methods the processing of the sample prior to detection of the target molecule represent a major time-consuming step of the detection. The reduction in sample handling steps of course also reduces the risk of errors or mistakes that may occur during sample preparation or amplification.

In particular embodiments of the methods of the invention however, one or more additional steps wherein the target molecule is amplified or processed in some other way (purified, enriched) prior to performing detection are envisaged.

Advantages of performing the detection methods of the present invention on a target after amplification is that the sensitivity of the methods is further improved. In some situations, this will also apply to purification steps that e.g. remove components interfering with the detection.

Another advantage of including a step comprising the amplification of the target in the sample is that during amplification or other processing of the target molecule, this target molecule may be labelled or tagged. In particular embodiments, methods of the present invention envisage a processing step (e.g. target amplification), whereby a tag is introduced onto the target that can serve as the recognition part for a target-specific probe. This is especially useful where the methods of the present invention are to be applied to targets for which no two target-specific probes binding to different parts of the target molecule exist or can be prepared. As mentioned above, particular tags for use in the inventions are tags that are one member of a specific binding pair, while at least one target-specific probe that is used for detection is the other member.

In methods of the present invention, the target can be bound by a first and a second target-specific probe. The (first and second) probes specific for the target molecule used in the methods of the present invention may be any molecule capable of specifically binding to part of the target molecule. Alternatively, the target molecule is provided with a tag and the target-specific probe is a probe which binds specifically to the tag attached to the target. When referring to binding of a probe to a target or a tag, both covalent and noncovalent binding is envisaged, and depends on the nature of the probes and target. Typical examples of binding include hybridisation and antibody-antigen interaction. The target-specific probes suitable for use in the context of the present invention include molecules selected from the group consisting of DNA, RNA, polypeptides, antibodies, antigens, carbohydrates, proteins, peptides, drugs, viruses, polysaccharides, lipids, lipopolysaccharides, glycoproteins, lipoproteins, nucleoproteins, nucleic acids, oligonucleotides, immunoglobulins, albumins, hemoglobins, cell-surface molecules, microorganisms, fragments, portions, components or products of microorganisms, and molecules smaller than 20 kDa.

Typically, the target-specific probes will correspond to one member of a specific binding pair, such as one member of the following pairs: biotin and (strept)avidin, an antigen and an antibody, a hapten and an antibody, a carbohydrate and a lectin, complementary nucleotide sequences, complementary peptide sequences, a ligand and a receptor, an enzyme cofactor and an enzyme, and an enzyme inhibitor and an enzyme. Moreover, derivatives or analogs of such binding pairs may also be used, as long as the (physical or chemical) binding remains specific. For instance, instead of the natural antigen of an antibody, a variant or part of this antigen may also be used as a specific binding pair member (and thus as (part of) the target molecule or of a target-specific probe), as long as it is specifically recognized by the other member. Similarly, oligonucleotides may be used that are not 100% complementary, but are still capable of hybridizing under suitable stringency conditions (e.g. with one or two mismatches).

In particular embodiments the methods of the present invention may need to discriminate between two closely related target molecules. It will be understood by the skilled person that in these embodiments, the specificity of the target probe for the target (or tag) is essential and non-specific or low-stringency binding with the target molecule should be excluded. For instance, where the methods of the present invention are used for the discrimination between target oligonucleotides carrying a single nucleotide polymorphism (SNP) and target oligonucleotides not carrying the SNP, hybridization conditions need to be strict and only perfect matches of the specific binding pairs can be allowed.

The target-specific probes used in the methods of the present invention are typically chosen in function of the target molecule to be detected. For instance, where the target molecule is a nucleic acid, the first and second target-specific probes will typically be complementary or partly complementary nucleic acids, each capable of specifically hybridizing with a different part of the target molecule. When the target molecule is a protein, it is particularly envisaged that the first and second target-specific probes are antibodies directed against a different epitope of the target which are specific for the target. However, other combinations are also envisaged, e.g. the use of antibodies directed against different parts of a target which is a nucleic acid.

Methods of the present invention envisage the use of one or more first and one or more second target-specific probes. The nature of the one or more first target-specific probes and the one or more second target-specific probes used for the detection of the target molecule may be the same or different. Both the first and second target-specific probes may be oligonucleotides, or may be antibodies. In alternative embodiments, the first and second target-specific probes used to detect the target molecule belong to a different category. For instance, one probe is an oligonucleotide and the other is an antibody. Or one probe is biotin and the other is an antibody. In principle, any combination of probes is envisaged, as long as the two different target-specific probes can bind simultaneously to the target without interfering.

Methods of the present invention may involve the detection of one or more targets. Typically, methods of the invention are applied for the detection of a specific target, and the SE(R)RS labelled particles will comprise one or more copies of the same target-specific probe directed against this target. Alternatively however, the SE(R)RS labelled particles can comprise different probes directed against the same target. Additionally or alternatively, the target may encompass a class of molecules and the first and second target-specific probes can be directed against different versions of the target, all representing the same 'common' target. For instance, where the object of the method is detecting a bacterial contaminant, and the nature of the contaminant is not critical, the methods of the invention may comprise the use of different first and second target-specific probe, while only using one type of label.

The attachment of the target-specific probes to the SE(R)RS labelled particle can be in different ways. Typically, the particles, the target-specific probes or both are functionalized. For instance, oligonucleotides functionalized with alkanethiols at their 3'-termini or 5'-termini readily attach to gold nanoparticles. Methods of attaching e.g. 3' thiol DNA to flat metal surfaces may also be used to attach oligonucleotides to nanoparticles. The alkanethiol method can also be used to attach oligonucleotides to other metal, semiconductor and magnetic or dielectric colloids and to (nano)particles of other material. Other functional groups for attaching oligonucleotides to solid surfaces include e.g. phosphorothioate groups and substituted alkylsiloxanes. Oligonucleotides terminated with a 5' thionucleoside or a 3' thionucleoside may also be used for attaching oligonucleotides to particles, as well as e.g. disulfides, carboxylic acids aromatic ring compounds, sulfolanes, sulfoxides, isonitriles, silanes, aromatic carboxylic acids, aldehydes, alcohols and methoxy groups and rigid phosphates. Those skilled in the art recognize a large variety of methods by which nucleic acids, antigen, antibodies, proteins, peptides, small molecules, carbohydrates or any target-specific probe can be bound directly or indirectly to particles. For example, linkers may be used to attach the specific binding member to the particle.

In methods of the present invention, SE(R)RS labelled particles are bound to one or different targets by way of target-specific probes. Typically, each particle will comprise more than one target-specific probe. This increases the probability of a particle being bound to the target. Moreover, in practice, most methods for attaching probes to the surface of a particle are based on physical properties and/or functional groups occurring over the entire surface of the particle, such that, in these methods, more than one probe will be bound to the particle. For instance, when applying procedures described in U.S. Pat. No. 6,506,564, a 13 nm gold nanoparticle will have about 110 nucleotide strands attached to it (see US2004/086897). Using bigger nanoparticles will result in a larger number of probes, as described in the prior art. In order to avoid that multiple targets bind to one particle (thereby countering the amplification effect), it may be desirable to make use of particles comprising only one or a limited number (e.g. 50 or less, 20 or less, or 10 or less) of target-specific probes each on their surface.

The SE(R)RS labelled particles used in methods of the present invention are any particles comprising, in addition to the first target-specific probe(s), two or more SE(R)RS labels. According to one embodiment the SE(R)RS labels are bound directly or indirectly to the surface of the particles. Alternatively, the particles are liposome shells comprising SE(R)RS labels in their core, which are released upon lysing (as described in Zayetseva et al. (Lab Chip, 2005, 5:801-811)).

In particular embodiments, the particles used in methods of the present invention are nanoparticles, to which SE(R)RS labels are either directly or indirectly attached. The SE(R)RS labelled nanoparticles used in particular embodiments of the methods of the invention are particles of 1-100nm comprising one or more SE(R)RS labels. Typically the particles comprise a core and a surface. While the nature of the material of the particles is not critical it should allow the binding of labels and probes to its surface. The material of the core of the particles need not necessarily be identical to the material of the nanoparticle surface. Optionally, the nanoparticles may be hollow. According to a particular embodiment, however, the labelled nanoparticles are solid particles entirely made of the same material. Materials that may be used for the nanoparticles (or their surface) include, but are not limited to, polymers, silicon, and metals. According to a particular embodiment the nanoparticles are of a noble metal such as gold, silver, platinum, copper or combinations or alloys thereof. The shape of the nanoparticles is not critical for practicing the methods of the invention, for instance spherical as well as needle-like particles may be used. According to a particular embodiment, spherical nanoparticles are used.

According to a particular embodiment of the invention, the SE(R)RS labelled nanoparticles are silver particles, or particles with a silver surface. According to another particular embodiment, the SE(R)RS labelled nanoparticles are gold particles or particles with a gold surface.

In the methods of the present invention, the labelled nanoparticles are typically provided in a solution, e.g. a colloidal solution.

SE(R)RS labelled particles used in the methods of the present invention can be characterized in that they comprise two or more SE(R)RS labels. The SE(R)RS labels may be any label or dye capable of being detected by SERS and/or SERRS methods. These include both fluorescent and non-fluorescent labels. SE(R)RS labels are well known in the art. A non-exhaustive list of examples include ROX, rhodamine 6G, HEX, FAM, TET, Cy3, Cy5, Cy3.5, TAMRA, TRIT (tetramethyl rhodamine isothiol), xanthines, succinylfluoresceins, N,N-diethyl-4- (5'-azobenzotriazolyl)-phenylamine, 4-(4-Aminophenylazo)phenylarsonic acid monosodium salt, arsenazo I, basic fuchsin, Chicago sky blue, direct red 81, disperse orange 3, HABA (2-(4-hydroxyphenylazo)-benzoic acid), erythrosin B, trypan blue, ponceau S, ponceau SS, 1,5-difluoro-2,4-dinitrobenzene, cresyl violet, aminoacridine and p-dimethylaminoazobenzene. These and other Raman-active molecules can be obtained from commercial sources. The skilled artisan will realize that the SE(R)RS labels that can be used in the context of the invention are not limited to those disclosed herein, but may include any known SE(R)RS label that can be attached directly or indirectly to a particle or included in the core of a particle for generation of the SE(R)RS labelled particles according to the methods of the invention.

Depending on the nature of the detection method envisaged, the two or more SE(R)RS labels present on or in the particle in the methods of the present invention are either identical or different. Where the goal of the methods of the invention is sensitive detection, the SE(R)RS labels typically will be identical or have identical optical properties so as to allow amplification of the signal of the target and increase sensitivity of the detection. Accordingly, the step of measuring the SE(R)RS spectrum of the two or more SE(R)RS labels adsorbed to the SERS active surface encompasses detecting the one SE(R)RS spectrum of the SE(R)RS label, whereby the SE(R)RS spectrum is amplified by the presence of multiple identical SE(R)RS labels in the detection area.

Alternatively, methods of the present invention can be used to allow a more accurate detection, by ensuring that the signal can be detected in two different ways. Accordingly, another aspect of the invention is the provision of methods for detecting a target in a sample, which ensure a more accurate detection. In these methods of the invention, the two or more SE(R)RS labels on the nanoparticles comprise at least two labels with distinct SE(R)RS properties. This allows the detection of one target molecule through at least two different SE(R)RS spectra. In this way, a "double" detection is ensured, improving accuracy of detection.

The at least two SE(R)RS labels with different optical properties may be present in equal amounts or in different amounts (e.g. 90% of one SE(R)RS label, 10% of another), and the respective strength of the signals may be used for standardization purposes.

Accordingly, the step of measuring the SE(R)RS spectrum of the two or more SE(R)RS labels adsorbed to the SERS active surface encompasses detecting the SE(R)RS spectrum of each of the SE(R)RS labels, whereby the detection of the different SE(R)RS spectra provides an internal control of the detection of the target.

It will be understood that combinations of the above embodiments are equally envisaged. Accordingly, it is possible to ensure both an amplification of the target signal by providing a particle which comprises more than one of the same SE(R)RS label, while at the same time ensuring a "double" signal, by introducing SE(R)RS labels with different optical properties on/in the particle.

Where the SE(R)RS labelled particles used in methods of the present invention are nanoparticles to which the two or more SE(R)RS labels are bound, the two or more SE(R)RS labels may be bound directly or indirectly. In particular embodiments of the methods of the present invention, the SE(R)RS labels are bound directly to the nanoparticle surface. Direct attachment of the SE(R)RS labels to the nanoparticles can be ensured in different ways. According to a particular embodiment, the direct attachment of the labels to the nanoparticle surface is via covalent binding. According to a further specific embodiment, the covalent binding is by means of a functional group such as a thiol group. Direct attachment of a SE(R)RS label to the nanoparticle surface can also be achieved by using e.g. benzotriazole dyes. According to specific embodiments of the invention, the SE(R)RS label has a benzotriazole moiety for covalent attachment to the nanoparticle surface. Attachment of the SE(R)RS label to the particle surface can also be ensured by means of electrostatic interaction.

In an alternative embodiment of the methods of the invention, the SE(R)RS labels are attached indirectly to the nanoparticles, by means of a support or linker molecule. The nature of such a support or linker molecule may vary, it may e.g. be a polymer, a sugar, a nucleotide or oligonucleotide, a protein, a lipid, and so on. These molecules may be natural or synthetic. However, the support or linker molecules should not interact with the target molecule to be detected, or interfere with the binding of the target-specific probe to the nanoparticle or with the binding of the target-specific probe to the target molecule. Typically, attachment of SE(R)RS labels to the nanoparticles by means of a linker molecule may be envisaged when the labels can not be directly attached to the nanoparticle surface. Linker molecules may be attached to the nanoparticles in a similar way as probes are attached to the particles (see above). The SE(R)RS labels may be attached to the linker or support molecule, or attached or associated with them, e.g. through electrostatic interaction. The linker molecules may each carry one SE(R)RS label, or more than one SE(R)RS label.

In yet an alternative embodiment of methods of the present invention it is envisaged that the SE(R)RS labels are bound indirectly to the nanoparticle, through the binding to one or more first target-specific probes which probes are bound to the particle. Again, the SE(R)RS labels may either be covalently attached to the target-specific probes, or attached or associated with them, e.g. through electrostatic interaction. In general, all of the probes on the nanoparticle will be labelled to obtain a stronger amplification (where the methods of the invention encompass the use of multiple identical SE(R)RS labels) or a higher specificity (where the methods of the invention encompass the use of different SE(R)RS labels, see below). However, it is envisaged that not all of the probes need to be labelled.

Where the SE(R)RS labels are bound to the probes instead of the particle itself, the SE(R)RS labelled target-specific probes are typically bound covalently on the nanoparticle surface so as to form a SE(R)RS labelled nanoparticle. According to particular embodiments, the covalent binding of the probes to the nanoparticle is through a thiol group. Typically, this thiol group is on the target-specific probe. The one or more first target-specific probes may each carry one SE(R)RS label, or more than one SE(R)RS label.

Combinations of the different embodiments of the SE(R)RS labelled nanoparticles described above are also envisaged for use in the methods of the present invention. Thus, a nanoparticle may carry SE(R)RS labels directly on its surface and/or attached to the first target-specific probes and/or attached to a linker or support molecule.

According to yet another aspect of the present invention, methods of detection are provided which allow multiplexing, i.e. the simultaneous detection of different targets in one sample. For this aspect of the invention, for each target, nanoparticles are used comprising one or more target-specific probes and two or more of the same SE(R)RS labels. Within one assay, the different particles (each carrying a target-specific probe specific for one target) can be combined for simultaneous detection of different targets.

Methods of the present invention envisage the detection of a target, by binding with a particle comprising more than one SE(R)RS label. To ensure accurate detection, the methods encompass the separation of the SE(R)RS labelled particles bound to the target and those not bound to the target, as the latter will interfere with the detection.

In methods of the invention described above, this is ensured by simultaneous binding of the target to a multiply labelled particle and to a manipulatable particle. Examples of manipulatable particles include, for example magnetic particles which can be manipulated by a magnetic field, (di)electric particles or electrets (e.g. polymer particles) which can be manipulated with an electric field, glass or polystyrene beads which can be manipulated by so-called optical tweezers, or particles which can be separated by size using a mechanical filter. Removal of the target-bound SE(R)RS labelled particles from the SE(R)RS labelled particles not bound to the target can then be ensured by applying a separation force. The nature of the separation force will typically depend on the nature of the manipulatable particles, e.g. by applying a magnetic field, magnetizable or magnetic particles can be manipulated. Similarly, an electric field can be applied to actuate (di)electric particles. Alternatively, optical trapping or mechanical filtering may be applied to separate the target-bound SE(R)RS labelled particles from the labelled particles not bound to the target molecule.

According to a specific embodiment, the manipulatable particles are magnetizable or magnetic particles. The nature of the material of the magnetizable or magnetic particles used in the methods according to the above-described aspect of the invention is not critical. It may be any material that is magnetic or can be magnetized during the performing of the detection method. Thus, the magnetic particles may be permanent or temporary magnets, made of magnetic metallic elements, composites (e.g. ceramic or ferrite magnets, alnico magnets, resins with magnetic powder), rare earth metals and the like. According to a particular embodiment, the magnetic particles are microparticles, i.e. of micrometer size. According to another embodiment, the magnetic particles are nanoparticles. Typically, the magnetic particles are provided in solution.

According to another particular embodiment, the manipulatable particles are dielectric particles. The nature of the material of the dielectric particles used in the methods according to the above-described aspect of the invention is not critical. Typical examples include plastic or synthetic polymer particles. Dielectric properties may be (quasi-)permanent or induced. According to a particular embodiment, the dielectric particles are microparticles, i.e. of micrometer size. According to another embodiment, the dielectric particles are nanoparticles. Typically, the dielectric particles are provided in solution.

The manipulatable particles are bound to the target in methods of the invention through a second target-specific probe. The nature of the second target-specific probe is not critical. Generally however, the nature of the second target-specific probe is similar to that of the first target-specific probe. Methods for binding of a target-specific probe to a manipulatable particle are referred to described above.

Accordingly, methods according to the above-described aspect of the invention involve a separation step (step (d) in the methods described above). As indicated above, the nature of the separation step is linked to the nature of the manipulatable label. Different methods of separation are envisaged such as, but not limited to separation using a magnetic field, an electric separation step using an electric field, application of an electromagnetic field, e.g. using optical tweezers or other methods of physically separating molecules such as mechanical separation based on size (filtration, centrifugation). Magnetic separation for instance may be ensured by applying a magnetic field, either directly (e.g. using magnets) or indirectly (e.g. via an electric field) to the solution obtained after steps (a) to (c), such that the target-bound SE(R)RS labelled particles are physically separated from the non-target-bound SE(R)RS labelled particles, allowing a separate detection. Typically, the separation step (magnetic, electric, optical, filtering or otherwise) is performed in solution, although this is no prerequisite. Indeed, the use of manipulatable particles has the important advantage that it makes it possible to carry out the detection methods of the invention in solution. When detection methods involving probes are carried out in solution, less hybridization or binding time is necessary to allow complete hybridization or binding (at least partly due to the easier diffusion in solution, allowing better mixing of components), and the observed hybridization (or binding) is more homogenous.

In general, the separation step will involve movement of the manipulatable particles, e.g. from a contact area, where different reagents are brought together, to an area where detection is possible. These areas may be different physical entities or part of the same physical entity. Typically, a separation force (e.g. a magnetic or electric field, or optical tweezers) is applied such that the target bound SE(R)RS labelled particles are moved to/concentrated in a separate part of the reaction vessel in which step (c) has been performed, e.g. the bottom of the vessel. Optionally, unbound manipulatable particles may be moved as well (e.g. unbound magnetic or dielectric particles will also be moved when applying a magnetic/electric field). According to another, specific embodiment however, the manipulatable particles are not moved during the separation step. This may be the case when the particles are held in place and the other components (i.e. unbound nanoparticles and the like) are washed away. Particles can be held in place e.g. by magnetic or electric means, via optical means such as optical trapping, or by mechanical means such as through a mechanical filter.

According to a further aspect of the invention, methods are provided wherein the separation of the SE(R)RS labelled particles amplifying the signal that are bound to the target and those that are not bound to the target is ensured by binding the target to a solid surface and washing away the non-bound SE(R)RS labelled particles. The methods according to this aspect of the invention do not require the application of a separation force other than the washing step. Another advantage of the methods according to this aspect of the invention is that the binding of the labels on a solid SERS-active surface allows detection directly on a roughened surface, which in principle is more homogenous than a colloidal suspension, while no (silver) staining step is required. As detailed in the art, for instance in US2004/086897, for sensitive detection of SE(R)RS labels, a staining step is necessary. Even when SE(R)RS active nanoparticles are used to enhance the signal, a staining step is required to detect very small concentrations of analyte (< 1 nM), as the nanoparticle spacings are too large. The use of a solid surface as SERS active surface allows for sensitive detection without the need for a staining step.

According to this aspect of the invention, methods are provided for the sensitive and/or accurate detection of a target molecule using surface-enhanced (resonance) Raman spectroscopy (SE(R)RS), wherein the target is 'sandwiched' between SE(R)RS labelled particles comprising two or more labels and a solid surface. More particularly, the methods according to this aspect of the invention comprise the steps of:
(a) providing particles carrying one or more first target-specific probes capable of binding specifically to part of the target molecule, wherein the particles comprise two or more SE(R)RS labels;
(b) providing a SERS active solid surface carrying one or more second target-specific probes capable of binding specifically to another part of the target molecule;
(c) allowing the target molecule to be bound, in random order, by both the first target-specific probes of the particles and the second target-specific probes of the solid surface, thereby obtaining a "sandwiched" target molecule;
(d) separating the "sandwiched" target molecule from the unbound particles by washing away the unbound particles; and
(e) measuring the SE(R)RS spectrum of the two or more SE(R)RS labels bound to the solid surface.

Similarly to the methods described above, the order of steps (a) to (c) is not critical and may vary. Thus, in practice, the step of providing the (nano)particles (a) and providing the SERS active solid surface (b) may take place in this order, the reverse order, or may be simultaneous. Similarly, in step (c) the target molecule can be bound first by the probe on the labelled particle, first by the probe on the solid surface or simultaneously by both probes. The target molecule may even be bound by one of the probes (i.e. part of step (c)) prior to providing the particles or surface with the other probe. Thus, steps (a), (b) and part of (c), i.e. the steps prior to the obtaining of the 'sandwiched target molecule' can be performed in random order, as long as they result in obtaining a 'sandwiched target molecule' if the target molecule is present.

Similarly to the methods described hereinabove, the methods according to this aspect of the invention also encompass the use of particles comprising two or more SE(R)RS labels and first and second target-specific probes which ensure the 'sandwiching' of the target between the SE(R)RS labelled particle and the SERS active solid surface.

The nature of the target(s) and corresponding first and second target specific probes is identical for the methods according to this aspect of the invention, compared to the methods described above and embodiments described therefore are equally applicable.

The separation step (d) in the methods according to this aspect of the invention however does not involve the application of e.g. a magnetic or dielectric field, but, as the target-bound SE(R)RS labelled particles are held in place on the solid surface, the unbound target molecule and unbound particles can be washed away. As the solid surface is a SERS active surface, the SE(R)RS spectrum of the two or more SE(R)RS labels bound via the target to the solid surface can be detected directly after the separation/washing step.

According to this aspect of the invention, methods are provided wherein the target is bound to a solid surface which is a SERS active surface. Examples of solid surfaces which can be used as a SERS active surface and are equally suitable for attaching probes are known to the skilled person. In particular embodiments, the solid surface is a solid metal substrate, more in particular a noble metal surface such as a solid silver substrate or surface, or alternatively a solid gold substrate or surface.

The solid surfaces used in the methods according to this aspect of the invention comprise a second target-specific probe, for binding of the target. Suitable methods for attaching probes to a solid surface will depend on the nature of the solid surface and the probe, but are similar to those described above for the attachment of the first target-specific probe to the particle. Typically the target-specific probes will be covalently attached to the solid surface, e.g. through a thiol moiety.

The methods according to the different aspects of the invention described above optionally comprise a 'releasing step', i.e. a step whereby the SE(R)RS labels are released from the 'sandwiched' target molecule (i.e. the target molecule that is bound to the SE(R)RS labelled particles and the manipulatable particles or solid surface).

This release of the labels typically occurs in one of three ways: either the SE(R)RS labels are released from the bound nanoparticle, or the SE(R)RS labelled nanoparticles are (in their entirety) released from the target molecule, or the SE(R)RS labelled nanoparticles and the target molecule are released from the manipulatable particle or solid surface.

According to particular embodiments of the invention, methods are provided wherein the SE(R)RS labels are released from the target molecule by releasing them from the particles. Depending on the nature of the particle and/or the nature of the bond between particle and label, the release of the SE(R)RS labels is ensured in different ways. In particular embodiments of the methods described above, nanoparticles are used whereby the labels are attached to the surface of the nanoparticle either directly or indirectly (e.g. via a target-specific probe, or via a linker molecule). Where the SE(R)RS labels are attached to the nanoparticles indirectly, the labels may be released from the nanoparticle by either releasing the labels from the probes or linker molecules, and/or by releasing the labelled probes or linker molecules from the nanoparticle. According to a particular embodiment, the release of the SE(R)RS labels from the nanoparticles is done by using dithiothreitol (DTT). According to another particular embodiment, the release of the SE(R)RS labels from the nanoparticles is ensured by an enzyme. The nature of the enzyme will depend on the bond that needs to be broken. E.g. restriction enzymes can be used to cleave oligonucleotides (labelled target-specific probes or linker molecules) from the particles, or glycosidases may be used to cleave carbohydrate linker molecules. Protein- and lipid-cleaving enzymes can be used similarly, where the labels are bound to proteins or lipids, respectively. Other suitable methods for releasing the SE(R)RS labels from the nanoparticles include, but are not limited to changing salt concentration or pH of a buffer (e.g. where the labels are attached via electrostatic interaction). A pH shift may also be used when streptavidin is attached on the nanoparticle surface and the SERRS labelled probes are additionally biotinylated and thus attached to the streptavidin. This coupling can be disrupted by changing the pH, e.g. using commercially available kits.

According to further embodiments of the methods of the invention, particles are used which are liposomes comprising the SE(R)RS labels within their shell. Release of the SE(R)RS labels from the liposome shell can be ensured by liposome lysis, e.g. by a nonionic surfactant (such as described by Zaytseva et al. (see above).

According to the methods of the present invention, upon release of the SE(R)RS labels from the particles, a SERS active surface is provided so as to allow detection of the released labels. These methods of detection are described more in detail below.

According to further particular embodiments of the methods of the invention, the labels are released from the target by releasing the entire labelled nanoparticle from the 'sandwiched' target molecule. This is of interest where labelled nanoparticles are used as such in the detection of the SE(R)RS spectrum, i.e. where the particles provide at least part of the SERS active surface for the detection of the spectrum of the SE(R)RS labels (see below). In these embodiments of the methods of the invention, the release of the SE(R)RS labels from the target molecule is typically ensured by breaking the bond between the target molecule and the one or more first target-specific probes. Methods for ensuring the breaking of this bond will again depend on the nature of the interaction. For instance, where the first target-specific probes and the target molecule are nucleic acids, the release step encompasses breaking or reversing the hybridization. Such methods are well known in the art, and involve e.g. heating, or changing pH or salt concentration of the environment. Alternatively, the target-specific probe and/or the target may be cleaved with a restriction enzyme, which also results in the release of the nanoparticle from the target, without necessarily affecting the hybridization between target and target-specific probe. For this purpose target specific probes comprising a restriction site can be used. Where the target and the target-specific probe are proteins, lipids or carbohydrates, the use of other enzymes is envisaged for the 'releasing step', e.g. glycosidases may be used to cleave carbohydrate probes, protein- and lipid-cleaving enzymes can be used similarly. In particular, immunoglobulin-cleaving enzymes are envisaged to be useful in cleaving antibody probes to release labelled nanoparticles bound to an antibody (or an antigen). Depending on how the nanoparticle is bound to the target molecule, other releasing methods may also be employed (e.g. DTT where a thiol group is used in the probe-target or probe-particle interaction). Similar methods may also be used to release the SE(R)RS labelled nanoparticles and the target molecule from the manipulatable particle or solid surface. This will typically be done by using an enzyme that cleaves the target molecule and possibly the second-target specific probe very close to the manipulatable particle (or solid surface).

According to particular embodiments of the methods of the present invention, an additional 'washing/separation' step is envisaged, after the release of the SE(R)RS labels from the target so as to separate the SE(R)RS labels from potentially interfering fractions (e.g. the non-labelled part of the sandwiched target molecule, unbound magnetic particles) prior to detection. Where the methods of the invention involve the use of magnetic particles, the additional washing/separation step can be ensured e.g. through another magnetic separation step. In a second magnetic separation step the unbound magnetic particles and the sandwiched target molecule which has been released from the SE(R)RS labels can be further removed (or physically separated) prior to detection. This may prevent interference of these components with the detection step. The same can be done for dielectric particles using an electric field, or via the application of other separation forces such as optical tweezers or a mechanical filter.

The methods according to the present invention are based on surface-enhanced (resonance) Raman spectroscopy (SE(R)RS) of the SE(R)RS labels which amplify the signal of the target.

To measure the SE(R)RS spectrum of the SE(R)RS labels used in the methods of the invention, a SERS active surface is provided, to which the labels can adsorb. According to a particular embodiment, the SERS active surface is a metal surface, more particularly a surface of a noble metal. According to a further particular embodiment, the SERS active surface is silver. According to an alternative particular embodiment, the SERS active surface is gold.

According to particular embodiments of methods of the present invention, the SERS active surface is at least in part provided by using, as SE(R)RS labelled particles, particles that have a SERS active surface. Accordingly, in these embodiments of the methods of the invention, step (f) is encompassed within step (a), in that the further provision of a SERS surface may not be necessary.

Examples of particles comprising a SERS active surface suitable for use in the methods of the present invention include metal nanoparticles, which may be naked metal or may comprise a metal oxide layer on a metal surface. They may include an organic coating such as of citrate or of a suitable polymer, such as polylysine or polyphenol, to increase their sorptive capacity. The metal particles comprising a SERS-active surface are typically colloidal nanoparticles which are generated in a controlled manner so as to be of a uniform and desired size and shape and as stable as possible against self-aggregation. Processes for preparing such colloids are well known (e.g. described in US Application No. 20050130163). Alternative methods of preparing nanoparticles are known (e.g. U.S. Pat. Nos. 6054495, 6127120, 6149868). Nanoparticles may also be obtained from commercial sources (e.g. Nanoprobes Inc., Yaphank, N.Y.; Polysciences, Inc., Warrington, Pa.). The metal particles can be of any size so long as they give rise to a SE(R)RS effect. Typically they have a diameter of about 4 - 50 nm, most particularly between 25 - 40 nm, depending on the type of metal. Methods for obtaining metal particles having a diameter of 40 nm have been described such as described by Munro et al. (Munro et al., Langmuir, 1995, 11: 3712-3720).

According to further embodiments of methods of the invention, the step of providing the SERS active surface is ensured by adding (nano)particles comprising a SERS active surface. These may be the same or different in nature as the particles carrying the first target-specific probes used in the methods of the invention, but are not labelled.

As indicated above, the nanoparticles used as SERS active surface in the methods of the present invention typically need to be aggregated prior to the actual detection or measuring step.

According to a particular embodiment, aggregation is ensured by adding an aggregating agent prior to detection. This aggregating agent may be provided together with the nanoparticles with the SERS active surface, or before or after the nanoparticles with the SERS active surface. Aggregating agents are compounds that allow the nanoparticles to aggregate in complexes. The use of aggregating agents is well known in the art, and the nature of the aggregating agent is not limiting to the invention, although it is possible that some aggregating agents will allow a more reproducible sensitive detection than others. Typically, aggregation of the nanoparticles is achieved by addition of a polyamine. Suitable polyamines include monomeric or polymeric polyamines, such as spermine or spermidine, 1,4-diaminopiperazine, diethylenetriamine, N-(2-aminoethyl)-1,3-propanediamine, triethylenetetramine and tetraethylenepentamine. Another example of an aggregating agent is a solution of sodium chloride.

For the embodiments of the methods of the invention described above involving detection making use of a SERS active surface which is provided by (nano)particles in suspension, the detection can be carried out completely in solution. Upon release of the SE(R)RS labels from the target, detection of the SE(R)RS labels is performed in solution. Detection in solution has several advantages over detection on a solid surface. It has been found that the generated signal is stronger. Another advantage is that target binding is faster in solution.

The aggregation of e.g. silver particles is a much faster procedure than e.g. a silver enhancement procedure typically used in the prior art to make non SERS-active surfaces SERS active. Aggregation occurs in a matter of minutes, while a silver enhancement protocol (e.g. in scanometric detection) typically requires a number of hours.

According to specific embodiments of methods of the present invention described above, the SERS active surface is provided in the form of a solid substrate. Typically, at least the surface of the solid substrate is of a noble (Au, Ag, Cu, Pt) or alkali (Li, Na, K) metal. The metal surface may for instance be an etched or otherwise roughened metallic surface. Typically, the solid substrate is a solid metal substrate, more in particular a solid silver substrate.

According to the methods of the present invention, the SE(R)RS labels are contacted with a SERS active surface before their SE(R)RS spectrum is measured. Where the methods of the present invention encompass the sandwiching of the target between a labelled particle and a solid SERS active surface, the SE(R)RS labels are, by the binding of the target to the solid surface, directly brought in the vicinity of the SERS active surface and the SE(R)RS spectrum of the two or more (identical or different) SE(R)RS labels is measured directly on the solid SERS active surface. In addition, it is possible to add additional colloidal nanoparticles to further enhance the signal. Alternatively, or concommittantly, the SE(R)RS labels can be released from the particle, target molecule or solid surface to bring them into closer proximity to the SERS active surface. Similarly, in the methods of the invention whereby the SERS surface is at least in part provided by the labelled particles themselves, the labels are directly in the vicinity of the SERS active surface and can be detected. Where the methods of the present invention encompass the use of a label-comprising liposome and/or where the methods encompass the release of the labels from the particle, typically the released SE(R)RS labels are brought into contact with a SERS active surface in the form of a colloidal suspension of metal particles to enhance the detection signal.

As detailed above, methods of the present invention encompass both the detection of one or more targets with improved sensitivity and/or the detection of one or more target molecules whereby a more accurate detection is ensured by the detection of two or more different SE(R)RS signals or spectra.

According to a particular embodiment, excitation is done at a single wavelength. The SE(R)RS signal can then be detected at a single wavelength. Alternatively, the whole SE(R)RS spectrum can be detected. A single spectrum is measured of the mixture of SE(R)RS labels, but it consists of contributions of the different labels, and specific peaks belong to specific labels. According to another embodiment, excitation can be done at more than one wavelength. This means that multiple measurements need to be taken. For every excitation wavelength, detection may be done at a single wavelength (detection of a SE(R)RS signal) or for the whole spectrum (detection of a SE(R)RS spectrum). The performing of multiple measurements (using several excitation wavelengths) may ensure that each distinct label is detected properly. According to a particular embodiment, the SE(R)RS signal of each of the SE(R)RS labels is detected at the wavelength which is optimal for that SE(R)RS label. Alternatively, multiple excitation wavelengths can be used and detection can be performed at a single wavelength or multiple wavelengths, or the whole spectrum is detected.

According to methods of the present invention, the measurement of the SE(R)RS spectrum of the SE(R)RS labels is an indication of the presence and/or quantity of a target in a sample. Indeed, the intensity and the nature of the SE(R)RS spectrum detected can be correlated to the presence of one (or different) target molecule(s) in the sample. When using quantitative correlation, a calibration curve can be made first, to assess the nature of the correlation between SE(R)RS signal and amount of target molecule.

Particular embodiments of the methods of the invention further envisage the detection of controls or internal standards, which allow the identification of false positives or negatives and allow for standardization.

Typically, some methods of the present invention encompass the use of particles comprising multiple identical SE(R)RS labels so as to increase sensitivity of detection. Additionally or alternatively, particles comprising two or more different SE(R)RS labels are used in the detection of a target molecule, so as to obtain a second signal for detection, increasing accuracy of detection. Of course, it is possible to combine both aspects of the invention, e.g. by using particles comprising both multiple identical SE(R)RS labels and, additionally, a smaller number of a distinct SE(R)RS label.

Methods of the present invention enable the performance of multiplexing assays. Indeed, the presence of different target molecules can be detected in a sample simultaneously. Typically this implies the use of differently labelled particles (differentially detectable label for each target), though it is envisaged that for particular purposes, i.e. where the contribution of each target is not critical) that identical labels are used. In any case this implies the use of appropriate target-specific probes for each target molecule to be detected.

Detection of the SE(R)RS spectrum of one or more SE(R)RS labels as envisaged in methods of the present invention, may be done using devices known in the art, e.g. commercially available spectrophotometers. The surface-enhanced Raman emission signal may be detected by a Raman detector. A variety of detection units of potential use in Raman spectroscopy are known in the art and any known Raman detection unit may be used. An example of a Raman detection unit is disclosed e.g. in U.S. Pat. No. US 6002471. Other types of detectors may be used, such as a charge coupled device (CCD), with a red-enhanced intensified charge-coupled device (RE-ICCD), a silicon photodiode, or photomultiplier tubes arranged either singly or in series for cascade amplification of the signal. Photon counting electronics can be used for sensitive detection. The choice of detector will largely depend on the sensitivity of detection required to carry out a particular assay. Several devices are suitable for collecting SE(R)RS signals, including wavelength selective mirrors, holographic optical elements for scattered light detection and fibre-optic waveguides. Various options are discussed, e.g. in WO 97/05280.

According to a further aspect of the invention, devices and systems are provided which are particularly suited for carrying out methods of the invention. Typically, such devices comprise a source for each of the reagents used in the methods of the present invention, means for contacting the different reagents, means for ensuring the separation step and means for detection.

According to one embodiment, the devices of the invention comprise a source of labelled particles, and a source of manipulatable particles suitable for performing the methods of the invention and a reaction chamber linked to the sources of reagents. Typically in this embodiment the systems comprise a means for applying a separation force (e.g. a magnetic or dielectric field) to (at least part of) the reaction chamber.

According to a particular embodiment, the systems can be provided with (disposable) cartridges, comprising a reaction chamber which are connectable to the different sources of reagents of the system and/or comprising one or more of their own sources of reagents. According to one embodiment, the systems provided by the present invention comprise:
(a) a contacting container or reaction chamber (optionally as part of a disposable cartridge) for contacting a sample containing the target molecule or suspected of containing the target molecule with:
   - at least one source of (nano)particles carrying one or more first target-specific probes capable of binding specifically to part of the target molecule, wherein the (nano)particles comprise two or more SE(R)RS labels;
   - at least one source of manipulatable particles carrying one or more second target-specific probes capable of binding specifically to another part of the target molecule; which sources are optionally included within the system,
(b) means for applying a separation force or field;
(c) a detection container or detection window, optionally integrated in the contacting container (a); and
(d) means for detecting the signal generated by the two or more SE(R)RS labels after a magnetic separation step.

The nature of the means for applying a separation force is determined by the nature of the manipulatable particles used and the force/field they can be manipulated with. For instance, where magnetizable or magnetic particles are used as manipulatable particles, the means for applying a separation force or field is a means for applying a magnetic field. These are well known to the skilled person. Examples include, but are not limited to, permanent or temporary magnets, made of magnetic metallic elements, composites (e.g. ceramic or ferrite magnets, alnico magnets, resins with magnetic powder), rare earth metals and the like. The magnetic field may also be generated indirectly, e.g. by means of an electric field. Similarly, means for generating a (di)electric field are well known (e.g. an electric current generator). In another particular embodiment, the separation force can be applied using optical tweezers. According to yet another particular embodiment, the separation force is applied via mechanical means such as by a mechanical filter.

In alternative embodiments, systems of the present invention comprise a source of labelled particles and a reaction chamber with a solid SERS active surface, on which detection can be performed. The reaction chamber or contacting container is connected to at least one source of washing buffer and the means for applying a magnetic field is omitted. In these embodiments the contacting container is provided with a solid SERS active surface and the means for detecting the two or more SE(R)RS labels are positioned such that detection occurs on the solid SERS active surface.

According to a particular embodiment, systems can be provided with (disposable) cartridges, comprising a reaction chamber which are connectable to the different sources of reagents of the system and/or comprising one or more of their own sources of reagents. According to one embodiment, systems provided by the present invention comprise:
(a) a contacting container or reaction chamber (optionally as part of a disposable cartridge) for contacting a sample containing the target molecule or suspected of containing the target molecule with at least one source (optionally also incorporated within the system) of particles or nanoparticles carrying one or more first target-specific probes capable of binding specifically to part of the target molecule, wherein the particles or nanoparticles comprise two or more SE(R)RS labels, whereby the contacting container comprises a solid SERS active surface.
(b) a detection container or detection window, optionally integrated in the contacting container (a); and
(c) means for detecting the signal generated by the two or more SE(R)RS labels bound to the SERS active surface.

According to particular embodiments of the devices of the invention, the means for detecting the signal(s) generated by the two or more SE(R)RS labels comprises at least a light source, a filter and a detection means. The light source typically is a laser. The filter may be used to filter only the light of the relevant wavelength, either from input or output or both, to facilitate detection. The detection means may be any means capable of detecting a Raman signal, and may e.g. be a fiber optics device, a part of a spectrophotometer, and the like.

Optionally, the systems of the invention are provided with a means for processing the signal detected with the detection means. A typical example of such processing means is a computer, a computational module, or a computer program product in combination with a computational module.

As indicated above, the systems of the invention optionally are envisaged to be used in combination with optionally disposable or reusable cartridges. Accordingly, yet another aspect of the invention provides (disposable) cartridges, for use in a system for detecting the presence and/or quantity of a (or more than one) target molecule in a sample. Different cartridges are envisaged for performing the different embodiments of the methods of the present invention.

Where use is made of manipulatable particles, the cartridge comprises one or more of the following components:
(a) a reaction chamber or contacting container which can be subjected to a separation force such that components within the reaction chamber are separated into a first and a second zone within the contacting container
(b) a detection window placed such that detection occurs in one of both zones of the contacting container
(c) optionally, a set of sources with:
   - at least one source of (nano)particles carrying one or more first target-specific probes capable of binding specifically to part of the target molecule, wherein the (nano)particles comprise two or more SE(R)RS labels;
   - at least one source of manipulatable particles carrying one or more second target-specific probes capable of binding specifically to another part of the target molecule;
   - at least one source of additives serving in the detection;
(d) means for ensuring the provision of specified volumes of the fluids from the sources to the contacting container.

According to a specific embodiment of the cartridges according to this aspect of the invention, the contacting container comprises a separate detection zone, and the cartridge is provided such that a magnetic field allows the movement of magnetic particles to and from the detection zone. Typically, the cartridge has at least one window for detecting the signal generated in the reaction chamber, more particularly in the detection zone or chamber therein. The same or a different window serves to let incident (laser) light fall on the SE(R)RS labels (adsorbed to a SERS active surface) in the detection zone or chamber.

The provision of a detection zone may e.g. be achieved by separating the two zones within the contacting containers via a porous membrane within one physical entity. Alternatively, the detection zone is provided as a separate chambers (detection chamber) whereby movement of magnetic particles is possible from one chamber to the other.

According to one embodiment, the detection zone or chamber comprises a SERS active surface. According to another embodiment, a source of SERS active nanoparticles is connected to the detection zone or chamber. According to a further embodiment, in addition to the nanoparticle source, a source of an aggregating agent is connected to the detection zone or chamber.

The at least one source of additives serving in the detection may comprise any reagent that is necessary to facilitate the detection methods of the invention. According to one embodiment, a source of additives contains at least one buffer that may be used as a washing buffer. According to another embodiment, one source of additives is provided for provision of those compounds that can release the SE(R)RS labels from the 'sandwiched' target molecule, e.g. one or more enzymes, or dithiothreitol (DTT). Further potential sources of additives may contain reagents such as nanoparticles with a SERS active surface, or aggregating agents such as spermine. These embodiments are not mutually exclusive, and the cartridge may contain several of these sources of additives.

According to a further aspect of the invention, disposable cartridges are provided for use in a system for detecting the presence and/or quantity of a (or more than one) target molecule, which comprise:
(a) a reaction chamber or contacting container which comprises a solid SERS active surface,
(b) one or more detection windows placed such that detection occurs on the solid SERS active surface,
(c) optionally, a set of sources with:
   - at least one source of (nano)particles carrying one or more first target-specific probes capable of binding specifically to part of the target molecule, wherein the (nano)particles comprise two or more SE(R)RS labels;
   - at least one source of buffer;
   - optionally, a source of one or more second target-specific probes capable of binding specifically to another part of the target molecule for binding to the SERS active surface
(d) means for ensuring the provision of the fluids from the sources to the reaction chamber.

The at least one source of buffer may serve for the washing step, to remove unbound particles from the solid surface. Thus, the cartridge may further contain an outlet to remove the washing buffer comprising excess unbound target molecule and nanoparticles. Cartridges of the present invention may further have additional sources incorporated therein, e.g. a source of sample containing the target molecule or suspected of containing the target molecule. Typically, this sample will also be provided in fluid form, to ensure that it can be easily transported through the cartridge. Another example of an additional source is a source of internal standard reagents. This may for instance be a control sample with a known concentration of target molecule, to be able to (semi-) quantify the target molecule in the sample. Alternatively, it may be another control molecule. In particular embodiments, the source of control reagents is provided separately and in combination with separate sources of nanoparticles and magnetic particles with appropriate probes. Optionally, a separate reaction chamber is provided for the control..

The means for ensuring the provision of the fluids from the sources to the contacting (and/or detection container) can be various, e.g. flexible or rigid tubes, a conduit, nano- or microchannels etc., as long as it is possible to transport certain volumes of the sources through these means to the relevant container at the relevant time. Which container is relevant at what time depends on the nature of the source: the nanoparticles and magnetic particles, the sample and additives used prior to the separation step will typically be guided to the contacting container, to be brought in contact with each other. The additives used in the actual detection step, or in the release of the labels from the target molecule, will typically be provided to the contacting container (or detection container) in a later step, to allow detection of the SE(R)RS spectrum of the SE(R)RS labels.

### EXAMPLES

### Example 1: Detection and quantification of influenza-specific genes in a sample using SERRS with multiple labels.

Highly pathogenic avian influenza caused by certain subtypes of influenza A virus in animal populations, particularly chickens, poses a continuing global human public health risk. Direct human infection by the avian influenza A subtype H5N1 virus has been responsible for considerable human mortality recently, stressing the need for rapid and accurate diagnosis. Type A influenza viruses are subtyped on the basis of antigenic differences in the external glycoproteins, the hemagglutinins (HA) and the neuraminidases (NA). The present invention offers a novel, rapid and accurate approach to viral subtyping by using RT-PCR and SERRS-based detection of viral nucleic acids.

Viral RNA is extracted from clinical samples and cDNA complementary for viral RNA is generated using viral reverse transcriptase and random primers according to Wright et al. (1995), J. Clin. Microbiol., 33:1180-1184. Multiplex PCR is carried out with two sets of primers specific for the HA and NA genes of influenza virus subtype H5N1 (as described in "Recommended laboratory tests to identify avian influenza A virus in specimens from humans", WHO Geneva, June 2005), designed to yield PCR products of 219 and 616 bp, respectively. Amplified products are subsequently detected by SERRS.

Thereto, two target-specific probes are designed to be complementary to the 5' and 3' ends of the PCR products of the HA and NA genes, respectively. For each PCR product, one target-specific probe is attached to a magnetic particle (sequence: GCC ATT CCA CAA CAT ACA CCC for the HA PCR product; TTG CTT GGT CGG CAA GTG C for the NA PCR product). The other target-specific probe is labelled with a SERRS dye (a different dye for HA and NA probes), and attached to a gold nanoparticle, using the methods described in US2004/0086897 (sequence: CTC CCC TGC TCA TTG CTA TG for the HA PCR product, labelled with HEX (a SERRS dye); CCA GTC CAC CCA TTT GGA TCC for the NA PCR product, labelled with TET (a SERRS dye)). Briefly, this entails the use of standard phosphoramidite chemistry to incorporate the SERRS label in the target-specific probes, followed by cleavage, deprotection and purification of the probes. These oligonucleotides (extended with 10 adenine residues as a linker) are SERRS labelled and (propylthiol)-capped. Gold colloids are prepared by reduction of HAuCl₄ with citrate as described in Frens, Nature Phys. Sci., 241, 20 (1973) and Grabar, Anal. Chem., 67, 735 (1995). Briefly, aqueous HAuCl₄ (1 mM, 500 mL) is brought to reflux while stirring. Then, 38.8 mM sodium citrate (50 mL) is added quickly. The solution color changes from pale yellow to burgundy, and refluxing is continued for 15 min. After cooling to room temperature, the red solution is filtered through a 1 micron filter. The attachment of the labelled oligonucleotides to the gold nanoparticles is then done by mixing an aqueous solution of 17 nM (150 µL) Au colloids, with 3.75 µM (46 µL) thiol-capped oligonucleotides and allowed to stand for 24 hours at room temperature in 1 ml Eppendorf capped vials. This results in gold nanoparticles carrying multiple labels (through carrying multiple oligonucleotides with a SERRS label). As the labelled oligonucleotides for the detection of NA and HA are non-complementary and thus will not hybridise with eachother, it is possible to prepare a solution of colloids for each of the oligonucleotide probes and combine equal amounts of each of the two nanoparticle solutions shortly before use.

Both the gold nanoparticles and the magnetic particles are added to the sample potentially comprising one or both target DNA molecules. Each target DNA molecule hybridises to (is "sandwiched" between) the target-specific DNA probes comprising the gold nanoparticle and the target-specific probes comprising the magnetic particle. The HA PCR product and the NA PCR product are thus characterized by the presence of a magnetic particle and nanoparticles bound to the PCR product through their complementary target specific probe (see schematic picture in Figure 1, Ia).

The 'sandwiched' target-DNA is separated from the solution by magnetic force separation. The SERRS labels are then released from the gold nanoparticles. The thiolated oligonucleotides can be released from the gold nanoparticle surface by washing with a buffer containing dithiothreitol (DTT). This results in multiple SERRS labels per target DNA in solution (see Figure 1, step II), HEX labels for the HA target DNA and TET labels for the NA target DNA.

Silver nanoparticles together with an aggregating agent are added to the solution with SERRS labels, in order to be able to measure the SERRS spectrum (schematically depicted in Figure 1, step III). Thereto, the above prepared solution is mixed with 10 µl spermine tetrachloride (100 mM in water, freshly prepared) followed by 250 µl water and 250 µl citrate-reduced silver nanoparticles (prepared as described by Munro et al. (1995), Langmuir, 11:3712-3720). Adsorption of the SERRS labels to the silver colloids brings the HEX and TET dyes in close enough proximity to the metal surface for enabling SERRS detection while fluorescence is quenched. Immediately after mixing the SERRS spectrum is taken from the prepared solution using a LabRam system (Jobin Yvon) with an Argon laser providing excitation at 514.5 nm.

Accurate detection of very low concentrations of viral HA and NA genes is achieved by the multiple labelled SERRS method of the invention.

### Example 2: Detection and quantification of HIV in a sample using SERRS with multiple labels.

To detect the presence of Human Immunodeficiency virus (HIV), silver nanoparticles and magnetic particles are prepared with probes of a sequence that are complementary to a statistically unique 33-nucleotide sequence in the HIV *env* gene: 5' AGA AGA TAT TTG GAA TAA CAT GAC CTG GAT GCA 3'. The target-specific probe on the silver particle has the following sequence: 5' TTA TTC CAA ATA TCT TCT 3', the magnetic particle is provided with probes having the sequence 5' TGC ATC CAG GTC ATG 3'. The target-specific DNA probes comprise optically active labels (SERRS labels, here Cy3) and are attached to the surface of the silver nanoparticles via the addition of thiolated DNA to which a SERRS label is bound. This is done using the same methods as described in Example 1.

The silver nanoparticles and magnetic particles are added to the sample potentially comprising HIV and accordingly the target HIV sequence.

The target HIV sequence hybridizes to ("is sandwiched between") the target-specific DNA probes of both the Ag-nanoparticle and the magnetic particle. This is schematically depicted in Figure 2, Ia.

The sandwiched target-DNA is separated from the solution (comprising unbound silver particles) by magnetic separation. Subsequently, the SERRS labels are separated from the magnetic particle, by separation of the SERRS labelled silver nanoparticle from the magnetic particle. This is done by releasing the target from the target-specific probes by heat separation, such that the hybridized sequence denatured and separate from each other.

The Ag-nanoparticles with the SERRS labels in solution are then aggregated, via the addition of spermine. The SERRS spectrum of the labels is measured on the aggregated particles.

### Example 3: Detection and quantification of anthrax in a sample using SERRS with multiple labels on a solid surface.

In this example, gold nanoparticles modified with oligonucleotides are used to detect the presence of a particular DNA sequence hybridized on a solid substrate in a three-component sandwich assay format (Figure 3, Ia).

Gold particles are provided comprising a target-specific oligonucleotide, i.e. a sequence which is complementary to a sequence within the anthrax lethal factor, consisting of 15 bases: 5' ATC CTT ATC AAT ATT 3'.

A solid substrate (also of use as SERS active surface) is provided with a different target-specific probe by attaching a 3'-thiol-modified 15 base capture oligonucleotide to a silver surface: 5' TAA CAA TAA TCC CTC 3'. Both target specific probes are complementary to a 30-base target sequence: 5' GAG GGA TTA TTG TTA AAT ATT GAT AAG GAT 3'.

A sample potentially comprising anthrax is added to the solid surface. Thereafter, the gold particles are contacted with the solid silver surface. The anthrax target sequence present in the sample is cohybridized ('sandwiched') by the target-specific molecules on the solid surface and on the gold particles (Figure 3, Ia).

The unbound nanoparticles, pieces of DNA, and other components of the sample are subsequently washed away.

In view of the close proximity of the SERRS labels to the SERRS active surface their SERRS spectrum is measured, without the need for (silver) enhancement.

## Claims

1. A method of sensitive detection of a target molecule using surface-enhanced (resonance) Raman spectroscopy (SE(R)RS), comprising the steps of:
(a) providing labelled particles carrying one or more first target-specific probes capable of binding specifically to part of the target molecule, wherein the labelled particles comprise two or more SE(R)RS labels,
(b) providing manipulatable particles carrying one or more second target-specific probes capable of binding specifically to another part of the target molecule,
(c) allowing the target molecule to be bound, in random order, by both the one or more first target-specific probes of the labelled particles and the one or more second target-specific probes of the manipulatable particles, thereby obtaining a "sandwiched" target molecule,
(d) separating the "sandwiched" target molecule from the unbound labelled particles,
(e) optionally, releasing the SE(R)RS labels from the manipulatable particles,
(f) providing a SERS active surface to which the SE(R)RS labels can adsorb,
(g) allowing the two or more SE(R)RS labels to adsorb to the SERS active surface, and
(h) measuring the SE(R)RS spectrum of the two or more SE(R)RS labels adsorbed to said SERS active surface.

2. The method according to claim 1, wherein the labelled particles are labelled nanoparticles.

3. The method according to claim 2, wherein the nanoparticles are gold nanoparticles or silver nanoparticles.

4. The method according to claim 1, wherein the manipulatable particles are magnetizable or magnetic particles, and the separating in step (d) is done by magnetic separation.

5. The method according to claim 1, wherein the target molecule is a nucleic acid, the first and second target-specific probes are oligonucleotides and specific binding is through hybridisation.

6. The method according to claim 1, wherein the SE(R)RS labels are attached to the first target-specific probes.

7. The method according to claim 6, wherein each probe carries more than one SE(R)RS label.

8. The method according to claim 1, wherein SE(R)RS labels are attached directly to the particle surface.

9. The method according to claim 1, wherein the provided SE(R)RS active surface is silver.

10. The method according to claim 1, wherein the release of the SE(R)RS labels from the manipulatable particles is done by releasing the SE(R)RS labels from the labelled particles.

11. The method according to claim 1, wherein the SE(R)RS active surface is provided in the form of nanoparticles that are aggregated before the measuring step.

12. The method according to claim 11, wherein the SE(R)RS labelled particles provided in (a) are nanoparticles comprising a SERS active surface which provide at least part of the SERS active surface of step (f).

13. The method of claim 1, wherein the labelled particles are nanoparticles comprising a SERS active surface and wherein the release of the SE(R)RS labels from the manipulatable particles in step (e) is performed by releasing the SERRS labelled nanoparticles from the target molecule or by releasing the one or more second target-specific probes from the manipulatable particles.

14. The method according to claim 11 or 13, wherein an aggregating agent is provided before the measuring step, in particular a polyamine.

15. The method according to claim 1, wherein the two or more SE(R)RS labels have identical SE(R)RS properties.

16. The method according to claim 1, wherein the two or more SE(R)RS labels comprise at least two labels with different SE(R)RS properties and which comprises the step of detecting the SE(R)RS spectrum of each of the different SE(R)RS labels adsorbed to said SERS active surface.

17. The method according to claim 1, which further comprises the step of correlating the SE(R)RS spectrum to presence of the target molecule.

18. A disposable cartridge for use in a system for detecting the presence and/or quantity of a target molecule in a sample, comprising:
(a) a contacting container which can be subjected to a magnetic field such that magnetic particles can be moved from a first zone to a second zone within the contacting container,
(b) a detection window placed such that detection occurs in one of both zones of the contacting container,
(c) means for ensuring the provision of the fluids from sources to the contacting container; and
(d) optionally, a set of sources comprising:
- at least one source of labelled particles carrying one or more first target-specific probes capable of binding specifically to part of the target molecule, wherein the labelled particles comprise two or more SE(R)RS labels on their surface
- at least one source of magnetic particles carrying one or more second target-specific probes capable of binding specifically to another part of the target molecule,
- at least one source of additives serving in the detection.

19. The cartridge of claim 18, further comprising a source of sample.

20. The cartridge of claim 18, further comprising at least one source of internal standard reagents.

21. The cartridge of claim 18, wherein the at least one source of additives comprises at least one source selected from the following: nanoparticles with a SERS active surface, aggregating agents, enzymes, buffers, and dithiothreitol (DTT).

22. The cartridge of claim 18, wherein the contacting container comprises a SERS active surface.

23. A system for detecting the presence or amount of a target molecule, comprising:
(a) means for contacting a sample containing the target molecule or suspected of containing the target molecule in a removable cartridge with:
- at least one source of labelled particles carrying one or more first target-specific probes capable of binding specifically to part of the target molecule, wherein the labelled particles comprise two or more SE(R)RS labels on their surface;
- at least one source of magnetic particles carrying one or more second target-specific probes capable of binding specifically to another part of the target molecule;
(b) means for applying a magnetic field to the removable cartridge;
(c) means for detecting the signal generated by the two or more SE(R)RS labels after a magnetic separation step in said removable cartridge.

24. The system of claim 23, wherein the means for detecting comprises a light source and a filter.

25. The system of claim 23, further comprising means for processing the signal detected with the means for detecting.

26. A method of sensitive detection of a target molecule using surface-enhanced (resonance) Raman spectroscopy (SE(R)RS), comprising the steps of:
(a) providing labelled particles carrying one or more first target-specific probes capable of binding specifically to part of the target molecule, wherein the labelled particles comprise two or more SE(R)RS labels,
(b) providing a SERS active solid surface carrying one or more second target-specific probes capable of binding specifically to another part of the target molecule,
(c) allowing the target molecule to be bound, in random order, by both the first target-specific probes of the particles and the second target-specific probes of the solid surface, thereby obtaining a "sandwiched" target molecule,
(d) separating the "sandwiched" target molecule from the unbound labelled particles by washing away the unbound labelled particles, and
(e) measuring the SE(R)RS spectrum of the two or more SE(R)RS labels bound to the solid surface.

27. The method according to claim 26, wherein the SERS active solid surface is a solid metal substrate, in particular a silver substrate.

28. A disposable cartridge for use in a system for detecting the presence and/or quantity of a target molecule, comprising:
(a) a contacting container comprising a SERS active solid surface optionally carrying one or more second target-specific probes; and
(b) means for ensuring the movement of the fluids from different sources to the contacting container, and optionally,
(c) a set of sources comprising:
- at least one source of labelled particles carrying one or more first target-specific probes capable of binding specifically to part of the target molecule, wherein the labelled particles comprise two or more SE(R)RS labels on their surface;
- at least one source of buffer.

29. The cartridge of claim 28, further comprising a source of sample containing the target molecule or suspected of containing the target.

30. A system for detecting the presence or amount of a target molecule, comprising:
(a) means for contacting, within a removable cartridge, a sample containing the target molecule or suspected of containing the target molecule with
- at least one source of particles carrying one or more first target-specific probes capable of binding specifically to part of the target molecule, wherein the particles comprise two or more SE(R)RS labels;
- a SERS active solid surface carrying one or more second target-specific probes capable of binding specifically to another part of the target molecule;
(b) means to ensure washing of SERS active solid surface; and
(c) means for detecting the signal generated by the two or more SE(R)RS labels on said SERS active solid surface.
